# EUROPEAN PATENT SPECIFICATION

(11) **EP 1 633 427 B1**
(45) Date of publication and mention of the grant of the patent: **14.10.2009**
(21) Application number: 04742922.0
(22) Date of filing: 11.06.2004
(51) Int. Cl.: A61M 35/00

(54) **IMPROVED DISPOSABLE APPLICATOR FOR TOPICAL COMPOSITION**
VERBESSERTER EINWEGAPPLIKATOR FÜR TOPISCHE ZUSAMMENSETZUNG
APPLICATEUR JETABLE AMELIORE POUR COMPOSITION TOPIQUE

(30) Priority: 13.06.2003 US 460682
(43) Date of publication of application: 15.03.2006
(73) Proprietor: Medlogic Global Limited, Plymouth, Devon PL7 5BG (GB)
(72) Inventor: STENTON, Richard J., Horrabridge, Devon PL20 7QZ (GB)
(74) Representative: Atkinson, Peter Birch
(86) International application number: PCT/GB2004/002527
(87) International publication number: WO 2004/110545

(56) References cited:
- WO-A-00/71198
- GB-A- 2 185 880
- US-A- 3 847 151
- US-A- 5 658 084

## Description

### BACKGROUND OF THE INVENTION

### Field of the Invention

The invention is directed to single-use disposable applicators for the application of liquids. More particularly, it is directed to improved applicators and methods for applying adhesives, sealants and coatings, and particularly for the application of medical adhesives, sealants and coatings.

### Description of the Related Art

Disposable containers for small to medium quantities of reactive or medicated liquids tend to be based on a few concepts: bottles screw caps; mechanical rupture of a thin section of a container, such as in a glass ampoule with a fragile neck, plastic eye lubricant container with a twist off tab, Unicep^{™} dispenser with a snip off point, and like. Where application or spreading of the liquid is also required, there are fewer options available, such as fragile breakage of a glass container inside an applicator, or mechanical piercing of a film of foil seal to release the contained fluid. In the first case, such as in the DERMABOND^{™} applicator, the breakage of a glass ampoule poses a risk of use or patient exposure to broken glass. Piercing a film or foil requires a mechanism such as a screw or pin, and most designs require use of two hands or a seal that can leak and spill the enclosed liquid. One newer design uses a built in weakness in a sealed tube which when squeezed causes the weakness to fail, creating an opening through which the enclosed liquid is release.

Typical of the state of the art is the Duraprep^{™} applicator made by 3M whereby a lever is depressed causing a flexible applicator to deform and break an internal ampoule thereby releasing the enclosed fluid. Similarly the DERMABOND^{™} applicator made by Jams Alexander consists of a flexible tube which is compressed between thumb and forefinger to crush a thin-walled glass ampoule and release the fluid for dispensing though a porous component sealed into one end of the tube. These applicators expose the user or patient to the risk of contact with broken glass.

In the Prevail applicator, the tubular head of the applicator is depressed against the bottle causing a mechanism to pierce a foil seal on the top of the bottle and release the enclosed liquid. This applicator is prone to leakage in use and requires two hands to operate.

In the Hardwood Popule^{™}, gentle squeezing pressure on the tubular container causes hydrostatic pressure to build up throughout the tube and causes a rupture at the weakest point This weak point has been deliberately created by melting a thin spot in the wall of the tube. A foam applicator pad covers the weakness and as the fluid escapes it can be spread using the applicator pad. The mechanism to generate the weakness appears to be intrinsically variable since the pressure needed to release the fluid is variable, with some Popules^{™} requiring significant force, and others bursting in transit.

In summary, the above prior applicators are messy due to leakage, dangerous because of the presence of glass, require two hands to operate or are variable in performance. What is needed is an applicator which provides for an economical combined primary container and applicator, which protects the contents until the container is opened, yet is easy to open, and allows the contents to be readily spread over an extended area. In the case of medical products it should also allow for easy sterilization by conventional methods, and should be useful in packaging and delivering medical adhesives and sealants for the protection or treatment of mammalian tissue.

GB-A-2 185 880 discloses a liquid dispensing apparatus comprising a generally tubular body portion which holds the liquid to be dispensed and which terminates in a tip of narrower cross-sectional area than that of the container body portion. The tip is surrounded by an absorbent swab member and is adapted to be ruptured from the container body portion to allow egress of liquid from the container body into the swab member.

### BRIEF SUMMARY OF THE INVENTION

In accordance with the invention, there is provided an applicator comprising:
a reservoir adapted to contain a fluid;
a pad in fluid communication with the reservoir (108); and
a tab serving to seal the fluid reservoir at a breakable connection,
wherein the reservoir comprises a wall which is adapted to flex sufficiently under pressure from an operator's finger to force fluid from the reservoir into the pad, the tab includes a stem attached to the applicator at the breakable connection, the pad includes a channel for receiving said stem, the tab stem passes through the pad, and the breakable connection is formed of friable material such that bending the of the tab relative to the applicator causes breaking of said breakable connection to allow passage of fluid from the reservoir into the pad **characterised in that** said stem is slidably received in said channel and in that the tab is separated by pulling the stem through the channel.

### BRIEF DESCRIPTION OF THE SEVERAL VIEWS OF THE DRAWINGS

Many advantages of the present invention will be apparent to those skilled in the art with a reading of this specification in conjunction with the attached drawings, wherein like reference numerals are applied to like elements, and wherein:

**FIG. 1** is a perspective view of an applicator in accordance with the invention;

**FIG. 2** is a top view of the applicator of FIG. 1;

**FIG. 3** is a sectional view taken along line A-A in FIG, 2;

**FIG. 4** is a cross-sectional view taken along line B-B in FIG. 2; and

**FIG. 5** is a sectional view of an applicator in accordance with another aspect of the invention.

### DETAILED DESCRIPTION OF THE INVENTION

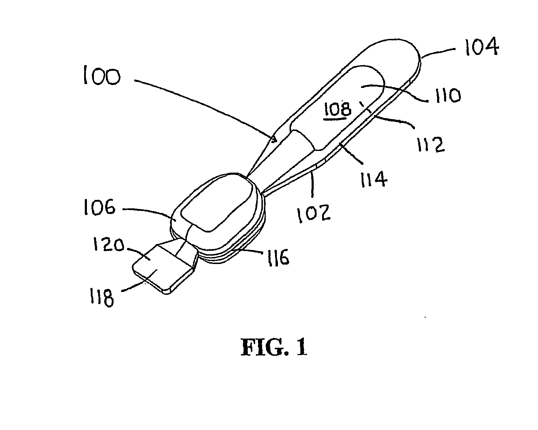

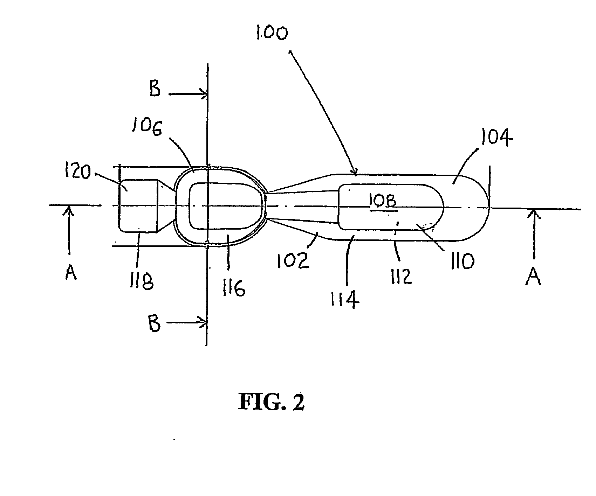

FIGS. 1 and 2 are perspective and top views of an applicator 100 in accordance with the invention. Applicator 100 comprises a generally elongate body 102 having a handle portion 104 and a dispenser portion 106. Handle portion 104 includes a reservoir 108 defined at its top and bottom ends by flexible walls 110 and 112, respectively. Reservoir 108 is adapted to contain a fluid, such as medical cyanoacrylate. A framing structure 114 radially surrounds reservoir 108 and provides rigidity to the applicator 100. Suitable materials for the reservoir 108 and framing structure 114 include high density polyethylene (HDPE), polyvinylchloride, polypropylene, polycarbonate, polytetrafluoroethylene (PTFE), FEP, and the like. When the fluid is medical cyanoacrylate, compatible materials such as HDPE and polypropylene could be used.

Dispenser portion 106 of applicator 100 comprises a pad 116, made from foam or other porous material, such as cotton, polyolefin foam, polyester polyurethane foam, polyether polyurethane foam, and so forth. Pad 116 is adapted to interface with the surface, such as the skin of a patient (not shown), to which fluid from the applicator 100 is to be applied and serves to spread the fluid for even application on the surface. Dispenser portion further includes a twist-off tab 118 having a twist-off handle 120, described in greater detail below.

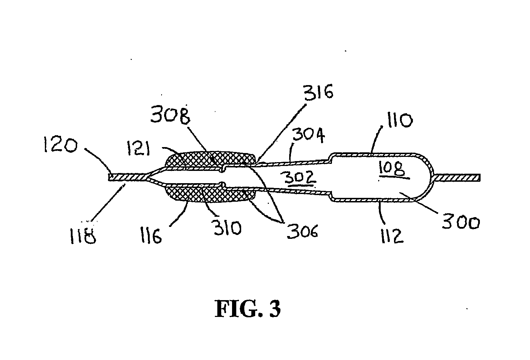

FIG. 3 is a cross-sectional view taken along line A-A in FIG. 2, and shows interior region 300 of reservoir 108 disposed in fluid communication with a conduit 302 extending into the interior of pad 116. Conduit 302 is defined by a generally tubular structure 304 which extends to a breakable connection 308 disposed within the interior of pad 116. One or more weld joints 306 attach pad 116 to tubular structure 304.

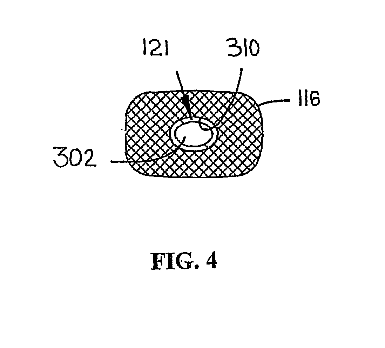

At the opposite side of breakable connection 308 and connected to tubular structure 304 via the breakable connection is tab stem 121 of twist-off tab 118. A channel 310 is formed in pad 116 for accommodating stem 121 and slidably receiving the stem therein. Breakable connection 308 is the only rigid connection between tab 118 and dispenser 100, and once this connection is severed, tab 118 can be readily separated from the dispenser by pulling stem 121 through channel 310. Preferably, channel 310, along with stem 121, are configured to be oval in cross section, as seen from FIG. 4, which is a cross-sectional view taken along line B-B in FIG. 2. The oval cross-sectional shape of channel 310 facilitates the closing of channel 310 by the preferably high density material of pad 116 once stem 121 is removed therefrom.

Reservoir 108, conduit 302 and twist-off tab 118 together form a hermetically sealed, sterilizable vessel 316 for holding fluid (not shown) within applicator 100. It is not necessary that tab 118 itself be capable of holding any fluid; therefore, although depicted as being hollow in nature, it is possible that stem 121 could be solid in design such that it will not contain any fluid.

Breakable connection 308 is designed as a weak point in the fluid-holding vessel 316, such that twisting and/or bending action relative to handle portion 114, applied by an operator's fingers to tab handle 120, will sever the twist-off tab 118 from the remainder of the vessel, breaking its integrity and permitting fluid flow into pad 116. This is accompanied, during use, by pressure applied to flexible walls 100 and 112 of reservoir 108 by the operator's fingers. The pressure forces the ejection of fluid from the reservoir 108 into pad 116, and onto the surface, such as the patients skin, to which the fluid is to be applied. The operator then slides the applicator 100, and specifically the pad 116, across the area onto which the fluid is to be applied, for example a wound in need of treatment with antibiotic, facilitating even and controlled application of the fluid onto the surface.

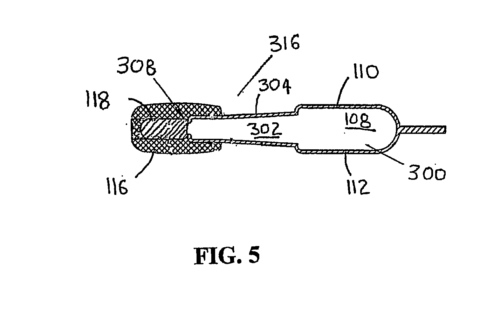

Alternatively, as shown in FIG. 5, tab 118 could be completely embedded in pad 116, and/or attached thereto, without the need for its removal to unseal the vessel 316. Rather, by simply bending pad 116 such that breakable connection 308 is at least partially broken or compromised, sufficiently to unseal vessel 316, fluid passage from the vessel into pad 116 can be realized. In such an aspect, a friable material, such as polypropylene, can be selected for at least the point of connection - that is, breakable connection 308 - in order to facilitate breakage by merely bending the pad at the breakable connection 308. For simplicity of design, the remainder of the applicator can be of the same friable material, with the weakest point being breakable connection 308. Further, in such aspect the tab 118 would not need to be slidably disposed in channel 310, but instead could be attached to pad 116, or otherwise contained therein.

Applicator 100 can be used to apply diverse types of fluids. It is particularly well-suited for use with sterilizable fluids for therapeutic applications to mammalian tissue, including topical liquids such as tissue adhesives for surgical applications, coatings and sealants. One candidate fluid is a cyanoacrylate prepolymer.

### Cyanoacrylate Adhesive Compositions:

The adhesive fluids that may be applied by the applicator of the present invention may be comprised of a wide variety of cyanoacrylate adhesive formulations. It is to be understood, however, that the present invention is not limited to any particular type of adhesive and any suitable medical (or non-medical) adhesive can be used. The reservoir may contain a stronger bonding and less flexible cyanoacrylate adhesive composition, such as *n*-butyl cyanoacrylate, or it may contain a more flexible tissue adhesive, such as an octyl or hexyl cyanoacrylate.

Preferably, the cyanoacrylate compositions used comprise cyanoacrylate prepolymer compositions that can be applied as a liquid/gel to the skin surface. Optionally, the cyanoacrylate prepolymers can include therapeutic agents such as analgesics, anti-inflammatory agents, antimicrobial agents, and the like.

Preferably, the polymerizable cyanoacrylate prepolymers comprise cyanoacrylate esters that, in monomeric form, are represented by formula I: 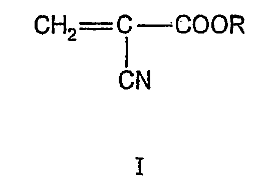 wherein
R is selected the group consisting of:
   alkyl of 1 to 10 carbon atoms,
   alkenyl of 2 to 10 carbon atoms,
   cycloalkyl groups of from 5 to 8 carbon atoms,
   phenyl,
   2-ethoxyethyl,
   3-methoxybutyl,
   and a substituent of the formula: 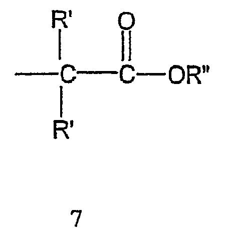
wherein
each R' is independently selected from the group consisting of: hydrogen and methyl, and
R" is selected from the group consisting of:
   alkyl of from 1 to 6 carbon atoms,
   alkenyl of from 2 to 6 carbon atoms,
   alkynyl of from 2 to 6 carbon atoms,
   cycloalkyl of from 3 to 8 carbon atoms,
   aralkyl selected from the group consisting of benzyl, methylbenzyl and phenylethyl,
   phenyl, and phenyl substituted with 1 to 3 substituents selected from the group consisting of hydroxyl, chloro, bromo, nitro, alkyl of 1 to 4 carbon atoms, and alkoxy of from 1 to 4 carbon atoms.

More preferably, in the cyanoacrylate esters of formula I, R is an alkyl group of from 2 to 10 carbon atoms including ethyl, *n*-propyl, iso-propyl, *n*-butyl, iso-butyl, sec-butyl, n-pentyl, iso-pentyl, n-hexyl, iso-hexyl, 2-ethylhexyl, n-heptyl, octyl, nonyl, and decyl. Mixtures of such compounds can also be employed as disclosed by Berger, et al., U.S. Patent No. 5,998,472.

It is to be understood that the term "polymerizable cyanoacrylate esters" refers to polymerizable formulations comprising cyanoacrylate monomers or polymerizable oligomers which, in their monomeric form, are preferably compounds represented by formula I as described above.

The polymerizable cyanoacrylate esters described herein rapidly polymerize in the presence of water vapor or tissue protein, and the *n*-butyl-cyanoacrylate bonds to mammalian skin tissue without causing histotoxicity or cytotoxicity.

Polymerizable cyanoacrylate esters are known in the art and are described in, for example, U.S. Patent Nos. 3,527,224; 3,591,676; 3,667,472; 3,995,641; 4,035,334; and 4,650,826 the disclosures of each are incorporated herein by reference in their entirety.

Optionally, the cyanoacrylate compositions applied by the present applicator can include a "biocompatible plasticizer". As used herein, the "biocompatible plasticizer" refers to any material which is soluble or dispersible in the cyanoacrylate composition, which increases the flexibility of the resulting polymeric film coating on the skin surface, and which, in the amounts employed, is compatible with the skin as measured by the lack of moderate to severe skin irritation. Suitable plasticizers are well known in the art and include those disclosed in U.S. Patent Nos. 2,784,127 and 4,444,933. Specific plasticizers include, by way of example only, acetyl tri-n-butyl citrate (preferably ∼20 weight percent or less), acetyl trihexyl citrate (preferably ∼20 weight percent or less) butyl benzyl phthalate, dibutyl phthalate, dioctylphthalate, n-butyryl tri-n-hexyl citrate, diethylene glycol dibenzoate (preferably -20 weight percent or less) and the like. The particular biocompatible plasticizer employed is not critical and preferred plasticizers include dioctylphthalate and C₂-C₄-acyl tri-n-hexyl citrates.

Optionally as well, the cyanoacrylate composition applied by the present applicator can include an "antimicrobial agent". As used herein, the term "antimicrobial agent" refers to agents which destroy microbes (i.e., bacteria, fungi, yeasts and viruses) thereby preventing their development and their pathogenic action.

Preferred cyanoacrylate compositions useful in the practice of this invention are also disclosed by Greff, et al., U.S. Patent No. 5,480,935, which application is incorporated herein by reference in its entirety. In a particularly preferred embodiment, the cyanoacrylate adhesive composition further comprises an antimicrobially effective amount of a compatible antimicrobial agent. Such compositions preferably comprise from about 0.1 to about 30 and preferably about 0.5 to 10 weight percent of the compatible antimicrobial agent either as a solution or as a suspension based on the total weight of the composition. Compatible antimicrobial agents are those which are either soluble or suspendable in the cyanoacrylate composition, which do not cause premature polymerization of the cyanoacrylate composition, which do not prevent polymerization of the cyanoacrylate composition when applied to mammalian skin, and which are compatible with the intended use including biocompatibility with the patient's skin. Suitable such compositions are disclosed in U.S. Patent No. 6,475,502, which discloses compositions of cyanoacrylate/povidone-iodine complexes, and U.S. Patent Application Serial No. 60/498,913 filed on August 29, 2003, which discloses compositions of cyanoacrylate esters and phenol.

The use of compatible antimicrobial agent in the compositions permits the agent to be released from the polymeric film thereby reducing microbial growth adjacent to the film.

Other medicaments suitable for use in conjunction with the cyanoacrylate compositions include corticoid steroids such as described by Greff, et al. in U.S. Patent No. 5,962,010 which is incorporated herein by reference in its entirety and analgesic compounds such as lidocaine. The former reduces inflammation whereas the latter reduces pain. Combinations of a steroid with an analgesic are also covered.

The above are exemplary modes of carrying out the invention and are not intended to be limiting. It will be apparent to those of ordinary skill in the art that modifications thereto can be made without departure from the scope of the invention as set forth in the following claims.

## Claims

1. An applicator (100) comprising:
a reservoir (108) adapted to contain a fluid;
a pad (116) in fluid communication with the reservoir (108); and
a tab (118) serving to seal the fluid reservoir (108) at a breakable connection (308),
wherein the reservoir (108) comprises a wall (110, 112) which is adapted to flex sufficiently under pressure from an operator's finger to force fluid from the reservoir (108) into the pad (116), the tab (118) includes a stem (121) attached to the applicator (100) at the breakable connection (308), the pad (116) includes a channel (310) for receiving said stem (121), the tab stem passes through the pad, and the breakable connection (308) is formed of friable material such that bending of the tab (118) relative to the applicator (100) causes breaking of said breakable connection (308) to allow passage of fluid from the reservoir (108) into the pad (116) **characterised in that** said stem (121) is slidably received in said channel (310) and **in that** the tab (118) is separated by pulling the stem (121) through the channel (310).

2. The applicator of Claim 1, wherein the channel (310) is oval in cross-section.

3. The applicator (100) of Claim 1, wherein the fluid is a sterilizable liquid.

4. The applicator (100) of Claim 1, wherein the fluid is a sterilzable tissue adhesive for surgical applications.

5. The applicator (100) of Claim 1, wherein the fluid comprises a cyanoacrylate ester adhesive.

6. The applicator (100) of Claim 1, wherein the fluid comprises butyl or octyl cyanoacrylate, or a mixture of butyl and octyl cyanoacrylates.

7. The applicator (100) of Claim 1 wherein the tab (118) has a twist-off portion (120) that protrudes from the pad.

## Patentansprüche

1. Applikator (100), der aufweist:
einen Behälter (108), der so angepaßt ist, daß er ein Fluid enthält;
ein Polster (116) in Fluidverbindung mit dem Behälter (108); und
ein zum Abdichten des Behälters (108) dienender Streifen (118) an einer zerbrechlichen Verbindung (308),
wobei der Behälter (108) eine Wand (110, 112) aufweist, die so angepaßt ist, daß sie sich unter dem Fingerdruck einer Bedienungsperson ausreichend durchbiegt, um Fluid aus dem Behälter (108) in das Polster (116) zu drücken, wobei der Streifen (118) einen Stutzen (121) aufweist, der an der zerbrechlichen Verbindung (308) an dem Applikator (100) befestigt ist, wobei das Polster (116) einen Kanal (310) zur Aufnahme des Stutzens (121) aufweist, wobei der Streifenstutzen durch das Polster hindurchgeht. und wobei die zerbrechliche Verbindung (308) aus sprödem Material besteht, so daß durch Biegen des Streifens (118) bezüglich des Applikators (100) ein Zerbrechen der zerbrechlichen Verbindung (308) verursacht wird, um den Durchgang von Fluid aus dem Behälter (108) in das Polster (116) zuzulassen, **dadurch gekennzeichnet, daß** der Stutzen (121) verschiebbar in dem Kanal (310) aufgenommen wird und der Streifen (118) abgetrennt wird, indem der Stutzen (121) durch den Kanal (310) gezogen wird.

2. Applikator nach Anspruch 1, wobei der Kanal (310) von ovalem Querschnitt ist.

3. Applikator (100) nach Anspruch 1, wobei das Fluid eine sterilisierbare Flüssigkeit ist.

4. Applikator (100) nach Anspruch 1, wobei das Fluid ein sterilisierbarer Gewebeklebstorff für chirurgische Anwendungen ist.

5. Applikator (100) nach Anspruch 1, wobei das Fluid einen Cyanoacrylatester-Klebstoff aufweist.

6. Applikator (100) nach Anspruch 1, wobei das Fluid Butyl- oder Octylcyanoacrylat oder ein Gemisch von Butyl- und Octylcyanoacrylaten aufweist.

7. Applikator (100) nach Anspruch 1, wobei der Streifen (118) einen von dem Polster vorstehenden abdrehbaren Abschnitt (120) aufweist.

## Revendications

1. Applicateur (100) comprenant :
un réservoir (108) adapté pour contenir un fluide;
un tampon (116), en communication de fluide avec le réservoir (108) ; et
une patte (118) servant à fermer de manière étanche le réservoir de fluide (108) au niveau d'une connexion cassable (308) ;
le réservoir (108) comprenant une paroi (110, 112), adaptée pour se fléchir de manière suffisante, lors de la pression exercée par le doigt d'un opérateur, pour entraîner le fluide du réservoir (108) dans le tampon (116), la patte (118) englobant une tige (121) fixée sur l'applicateur (100) au niveau d'une connexion cassable (308), le tampon (116) englobant un canal (310) pour recevoir ladite tige (121), la tige de la patte traversant le tampon, la connexion cassable (308) étant formée à partir d'un matériau friable, de sorte que le fléchissement de la patte (118) par rapport à l'applicateur (100) entraîne la cassure de ladite connexion cassable (308), pour permettre le passage de fluide du réservoir (108) dans le tampon (116), **caractérisé en ce que** ladite tige (121) est reçue de manière coulissante dans ledit canal (310) et **en ce que** la patte (118) est séparée en tirant la tige (121) à travers le canal (310).

2. Applicateur selon la revendication 1, dans lequel le canal (310) a une section transversale ovale.

3. Applicateur (100) selon la revendication 1, dans lequel le fluide est constitué par un liquide stérilisable.

4. Applicateur (100) selon la revendication 1, dans lequel le fluide est constitué par un tissu adhésif stérilisable destiné à des applications chirurgicales.

5. Applicateur (100) selon la revendication 1, dans lequel le fluide comprend un adhésif d'un ester cyanoacrylique.

6. Applicateur (100) selon la revendication 1, dans lequel le fluide comprend du cyanoacrylate de butyle ou d'octyle, ou un mélange de cyanoacrylates de butyle et d'octylc.

7. Applicateur (100) selon la revendication 1, dans lequel la patte (118) comporte une partie à rupture (120) débordant du tampon.
